(19) Europäisches Patentamt
European Patent Office
Office européen des brevets

(11)     EP 2 526 859 B1

(12)     **EUROPEAN PATENT SPECIFICATION**

(45) Date of publication and mention
of the grant of the patent:
**28.10.2020 Bulletin 2020/44**

(51) Int Cl.:
*A61B 5/0404* (2006.01)    *A61B 5/0428* (2006.01)
*A61B 5/04* (2006.01)

(21) Application number: **12168978.0**

(22) Date of filing: **22.05.2012**

(54) **Portable and wearable system for the acquisition, displaying, storage and proximal elaboration of an electrocardiographic signal (ECG), for recognising arrhythmic and ischaemic events, with remote transmission**

Tragbares System für die Ermittlung, Anzeige, Speicherung und Auswertung eines elektrokardiographischen Signals zur Erkennung von arrhythmischen und ischemischen Ereignissen mit einer Datenfernübertragung

Système portable pour l'acquisition, visualisation, stockage et une élaboration proximale d'un signal électrocardiographique pour reconnaître des événements arythmique et ischémique avec télétransmission

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.05.2011 IT MI20110957**

(43) Date of publication of application:
**28.11.2012 Bulletin 2012/48**

(73) Proprietors:
• **Pizzuto, Rodolfo**
  **24040 Zingonia (Bergamo) (IT)**
• **Politecnico Di Milano**
  **20133 Milano (IT)**

(72) Inventor: **POZZI, Giuseppe**
**22021 Bellagio (Como) (IT)**

(74) Representative: **Petruzziello, Aldo**
**Racheli S.r.l.**
**Viale San Michele del Carso, 4**
**20144 Milano (IT)**

(56) References cited:
**US-A- 5 876 351**     **US-A1- 2007 208 262**
**US-B1- 6 970 737**

## Description

**Field of the invention**

The present invention refers in general to the field of portable devices for the acquisition, displaying, storage, transmission and elaboration of biological signals on human subjects. More specifically, the invention concerns a system for the acquisition, displaying, storage, transmission and analysis of the electrocardiographic signal (ECG) acquired on medium and long length recordings, for recognising arrhythmic and ischaemic events.

**Background of the invention**

In modern societies of industrialised countries the main causes of death are neoplastic pathologies and cardiac pathologies. With particular reference to cardiac pathologies, they are divided into numerous classes, the most numerous of which are arrhythmia and ischaemia: an appropriate diagnosis made at the immediate onset of such pathological phenomena results in high success rates of the therapeutic treatment applied, with consequent reduction in deaths and improvement in the quality of life of patients.

Hence the importance of being able to identify and diagnose cardiac pathologies, both ischaemic and arrhythmic, correctly and quickly after their respective onset. Such identification takes place through the acquisition and elaboration of the electrocardiographic signal (ECG) of the subject.

The acquisition and transmission of ECG signals for subsequent elaboration has already been amply dealt with and covered by numerous patents.

For example, patent U.S. 5704351 to Mortara foresees the transmission of some signals (including ECG). However, such a system is aimed at just transmitting the signal from the acquirer to a proximal system, and does not consider a possible simultaneous storage in the acquirer for subsequent transmission.

Patent U.S. 5755230 to Schmidt et al. also does not provide any storage of the signals at the acquisition device, even if the patent considered the electroencephalographic signal (EEG).

Patent application US 2002/0115914 A1 does not indicate either the sampling frequency of the ECG signal or the relative quantization, and in particular it does not give the adequacy of such parameters with the existing recommendations on the subject (e.g. from the American Heart Association or American College of Cardiology).

Patent application US 2004/109992 A1 focuses just on the control of signal transmission, going from radio technologies that cover short distances to radio technologies that cover long distances: the types of signal under examination are not considered.

Patent application EP 1 629 769 A1 does not consider all 12 standard leads used in clinical practice, but focuses just on a non-clinical lead (fig. 8 of the patent).

Patent application EP 1 803 392 A2, which presents a single-use device, also does not consider clinical leads.

Patent application EP 2 082 683 A1 is able to consider just one lead at a time and does not provide simultaneous monitoring on many leads: moreover, it is not able to consider all the leads normally used in clinical practice.

Patent application EP 2 000 085 A2 to Politecnico di Milano, even if relative to an apparatus characterised by particularly small dimensions, considers exclusively a single lead and does not broach the simultaneous observation on many leads.

Patent application EP 1 188 412 A2 is able to consider many leads: however, it is aimed at monitoring patients with ischaemic pathologies, i.e. patients that have already undergone an initial screening. The maximum content in frequency in the electrocardiographic signal affected by from ischaemic pathologies is less than the maximum content in frequency in the electrocardiographic signal affected by other pathologies, like for example arrhythmic pathologies: an instrument not equipped with a suitable band pass could deform the alterations of the electrocardiographic signal due to some pathologies, like for example arrhythmic ones, making the latter actually difficult to identify, recognise and classify. The same system does not indicate the maximum number of leads that can be considered and whether it is able to acquire and elaborate all of the leads simultaneously, thus allowing the analysis of every single beat as observed simultaneously according to the 12 leads.

Again in patent application EP 1 188 412 A2 the capabilities of a mobile telephone and of possible comparable instruments are exploited just for the remote transmission of the signal: such capabilities are not used either for local elaboration (for example for calculating the instantaneous cardiac frequency, classifying arrhythmia, analysing the area subtended by the portion ST-T with respect to the base line) or for on-site displaying on the patient side of the trace: one only has to consider that the simple detachment of an electrode, which would invalidate the acquired trace, cannot be recognised apart from by the remote analysis centre. The absence of a valid telephone signal for the operation of the mobile telephone, moreover, would make any type of remote transmission for display and analysis impossible.

Patent application WO 2004/030756 A1, which also exploits Bluetooth or similar types of communication, uses just two electrodes, actually limiting the analysis to a single lead under examination.

Patent EP 0673233 provides an electrical coupling between the acquisition subsystem, which is in direct contact with the subject, and the elaboration subsystem powered by the electrical network at 220V. The same patent EP 0673233 does not provide the possibility of remote transmission of the acquired signals or local storage of historical recordings, or the possibility of carrying out elaborations, analyses and recognitions on site.

Patent US 5876351 does not provide the remote signals transmission, and nor does it provide the simultaneous detection of all of the main clinical leads. The display resolution, the quantization and the sampling frequency do not satisfy the recommendations of the relevant regulating bodies. Patent US 5876351, moreover, does not provide any type of elaboration, analysis, recognition carried out locally on the traces acquired. Patent US 5876351, finally, provides a limited duration of the recording as permitted by the memory of the device used.

Patent US 2007/0208262, correlated patents US 2007/0208232 and US 2007/0208233, the publication "A Multiparameter Wearable Physiologic Monitoring System for Space and terrestrial Applications", IEEE TITB, vol. 9, n. 3, Sept. 2005 by the same authors allow displaying of the traces only at the end of the acquisition session (paragraph [0064] in document US 2007/0208262). Document US 2007/0208262 and the correlated documents describe local displaying of just the parameter data, e.g. on a wrist watch shaped display (Fig. 9 of document US 2007/0208262), and do not allow the displaying, elaboration, and analysis in real time of the electrocardiographic traces. Moreover, document US 2007/0208262 and the correlated documents do not provide recognising in real time event and critical alterations that can suggest to the subject that they head straight for a treatment centre or a hospital, nor for this purpose are they able to indicate to the subject which treatment centre or hospital is nearest or quickest to get to.

US 6,970,737 discloses a method and an apparatus to remotely monitoring patients using a portable ECG device having a wireless communication interface and comprising a multi-lead, a multi-channel ECG monitor suitable for allowing a 24-hour surveillance and coupled with a communication device for communicating with a centralized facility (i.e. an hospital) and, further, comprising a GPS system connected to the wireless communication interface and indicative of the location of the patient; however, also said technical solution does not consider any local or proximal processing of the ECG signal in order to analyse and detect in real time any arrhythmic and/or ischaemic alteration and the GPS signal just give information and depends on the availability of the phone network.

US 8,763,351A discloses a diagnostic medical device which can only display the signal (one ECG leads, only) and does not perform any processing or analysis, neither locally nor remotely.

All of the systems described in the aforementioned documents have not proven to be completely satisfactory.

Normal long-lasting Holter recordings, for continuously monitoring the electrical activity of the heart, are not able to consider all of the clinical study leads (D1, D2, D3, aVr, aV1, aVf, V1, V2, V3, V4, V5, V6) but generally are limited to considering from 1 to 3 leads: this is because Holter recordings are carried out on subjects not as a first study but to get more information on situations already analysed with clinical studies that have considered all of the leads. In addition, Holter type systems tend to be larger in size and are not always able to transmit the signals in real time and simultaneously store them locally.

Therefore, it is of primary importance to be able to have an instrument suitable for the acquisition, displaying, storing, elaboration and transmission of the ECG signal, which is easy to use, of low weight and bulk, having good operating autonomy, with good professional characteristics comparable to those of professional equipment normally used in clinical and hospital environments.

The purpose of the invention is indeed to propose an instrument with the characteristics given above (easy use, low weight and bulk, good operating autonomy) that make it particularly suitable for use even in awkward contexts, for first aid, in the case of an emergency even with assistance of just paramedics, far from hospitals or first aid centres, even in the case of a lack of communication channels like for example a telephone line, mobile telephone network (GSM, UMTS), Internet, Wifi and similar.

Another purpose of the invention is to provide such an instrument suitable for obtaining all twelve clinical leads.

These purposes are accomplished according to the invention with the characteristics of the attached independent claim 1. Advantageous embodiments of the invention can be seen from the dependent claims.

The instrument according to the invention is suitable for carrying out the acquisition of an ECG signal, transmitting it to a proximal elaborating subsystem suitable for displaying, storing and carrying out the proximal first elaboration: the proximal elaborating subsystem is in turn connected to communication channels (like for example those belonging to the group including the telephone line, mobile telephone network, Internet, WiFi), and suitable for remotely transmitting the acquired signals, for a clinical evaluation by experts located even remotely with respect to the location of the subject under examination. The transmission to a proximal elaborating subsystem can be carried out in real time, or at the same time as the acquisition in "monitor" mode, or later on: in this last case the ECG signal must be stored temporarily at the acquisition subsystem and made available subsequently.

The present invention also carries out tasks of initial screening, suitable for a study of all 12 clinical leads that are essential for the correct diagnostic and therapeutic understanding of cardiological emergencies. The present invention is aimed at the analysis of ECG traces of patients who may not yet have been studied previously, e.g. because the pathological conditions came on extremely recently like for example in patients assisted by first aid.

The entire system is characterised by great ease of use that permits its use even by paramedics or first aid volunteers, without specific training, The entire system has a low weight, limited bulk, and its portability and wearability allow it to be used even in critical conditions and wherever the weight and size of the equipment are limiting factors (like for example athletes while playing their sport, orbiting stations, interplanetary travel and in space, hyperbaric chambers, submarines), or during clinical examinations (like for example the Master test, the strength test in general including those based on the use of an ergo-cycle, treadmill, other).

The entire system is also characterised by very low energy consumption: using some samples of the proposed system, it is possible to make a Picu (portable Intensive Care Unit) able to be used even in the case of disasters that strike an entire area, Indeed, since the system is also able to work on site and with extremely low energy consumption, a normal 12V car battery could allow the operation of monitoring of 12 beds for more than 24 hours uninterrupted,

The system according to the invention is made up of an acquisition subsystem and a proximal elaborating subsystem.

The acquisition subsystem is suitable for acquiring the ECG signals, preamplifying them, filtering them, amplifying them, converting them into digital format, storing them locally and transmitting them to the proximal elaborating subsystem. The acquisition subsystem keeps all of the properties of the analogic signals under examination unaltered without introducing alterations and it allows an analysis *a priori* of the subject, irrespective of the type of pathology presented, be it ischaemic, arrhythmic or of another kind, provided that it can be identified through an analysis of the electrocardiographic signal.

The proximal elaborating subsystem is suitable for carrying out tasks of displaying, storing, analysing, recognising and transmitting. Displaying takes place on the display of the same proximal elaborating subsystem: the proximal elaborating subsystem allows compact displaying of the signals and keeps the possibility of displaying the signal unaltered at the full sampling frequency.

Storage takes place in the memory of the proximal elaborating subsystem according to standard formats for storing ECG signals. The proximal elaborating subsystem is suitable for also allowing a comparison between the current recording and other recordings both of the same subject and of other subjects.

The proximal elaborating subsystem is suitable for carrying out a first local analysis of electrocardiographic signals, calculates the instantaneous cardiac frequency, and identifies the main alterations, both arrhythmic and ischaemic. The proximal elaborating subsystem is also suitable for calculating the current position through GPS signals and for calculating the distance from the current position to hospitals or treatment centres for which the geographical coordinates have been inserted and/or stored: this allows the operator or the subject himself to immediately identify the nearest hospital or treatment centre.

The proximal elaborating subsystem is suitable for using communication channels like for example telephonic wires, mobile telephone signals, Internet, Wifi to remotely transmit the signals acquired, to allow the subsequent analysis by specialists.

The proximal elaborating subsystem is also able, upon request from the user, through a panic button, to send a message to the mobile telephone of his doctor with the URL (Universal Resource locator) to access the trace that the same proximal elaborating subsystem will have sent to the dedicated Web server. In this way, the doctor can remotely check the possible electrocardiographic variations that the subject has undergone. In order to ensure suitable privacy, the doctor will have to insert a suitable enabling password to be able to access the functions of the dedicated Web server.

The proximal elaborating subsystem can consist of a system belonging to the group that includes PC, tablet PC, tablet, iPad, mobile telephone, smart-phone.

**Brief description of the drawings**

Further characteristics of the invention will become clearer from the following detailed description, referring to an example, and therefore not limiting, embodiment thereof, illustrated in the attached drawings, in which:

- Fig. 1 illustrates the entire system made up of the acquisition subsystem and the proximal elaborating subsystem;
- Fig. 2 is a block diagram of the electrocardiographic acquisition subsystem according to the invention, made up of an analogue module, a digital module, and a transmission module;
- Fig. 3 is a plan view from above of the control panel of the shell of the electrocardiographic acquisition subsystem according to the invention;
- Fig. 4 describes the information provided by the proximal elaborating subsystem.

**Detailed description of a preferred embodiment of the invention**

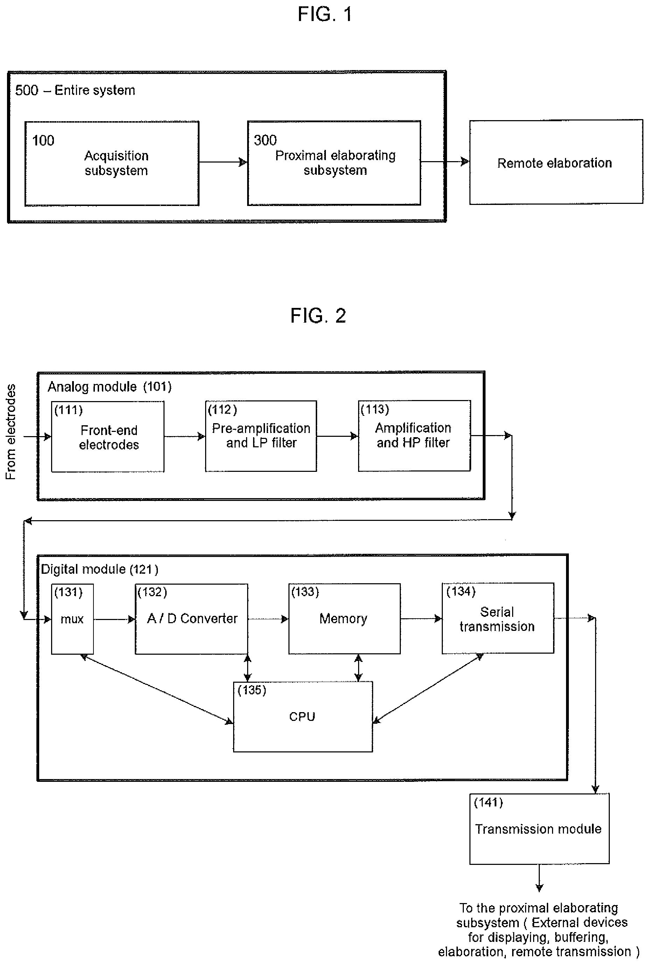

The system according to the invention, indicated in Fig. 1 by the block 500, is made up of an acquisition subsystem 100 and a proximal elaborating subsystem 300.

**Acquisition subsystem**

The electrocardiographic acquisition subsystem 100 according to the invention allows all twelve clinical leads to be examined.

The leads considered are: D1, D2, V1, V2, V3, V4, V5, V6. The number of electrodes considered is 10: from these it is possible to acquire the two leads D1 and D2 (which require the use of four electrodes) and the precordials V1, V2, V3, V4, V5, V6 (which require one additional electrode each).

Starting from the leads D1 and D2, it is then possible, through elaboration, to reconstruct the remaining clinical-interesting leads (D3, aVr, aV1, aVf), by exploiting the following formulae that are known in the literature:

$$D3 = D2 - D1;$$

$$aVr = - (D1 + D2) / 2;$$

$$aVl = D1 - (D2 / 2);$$

$$aVf = D2 - (D1 / 2).$$

The reconstruction operation, made possible by the proximal elaborating subsystem 300 after receiving the signals D1, D2, V1, V2, V3, V4, V5, V6 acquired by the acquisition subsystem, is immediate and does not require particular calculating power. Such a reconstruction occurs correctly and faithfully, i.e. the reconstructed leads are comparable with the corresponding leads that could be acquired directly on the subject, thanks also to the fact that the channels D1 and D2 are treated by the relative acquisition chains with an identical gain/amplification and a common base line: therefore, there are no particular alterations or artefacts introduced due to the reconstruction of the leads not acquired directly (D3, aVr, aVl, aVf), apart from to a minimal extent due to the minimal time jitter between D1 and D2,

The electrodes are positioned on the body of the subject according to normal clinical practice. Some of the electrodes are internally connected together through suitable circuits so as to constitute the neutral observation level or active mass.

With reference to the block diagram of Fig. 2, the acquisition subsystem 100 is made up of two main modules: an analogue module 101 and a digital module 121.

Although in the figure, for the sake of simplicity of representation, a single analogue module is shown, in practice there is one analogue module 101 for each of the eight clinical leads acquired D1, D2, V1, V2, V3, V4, V5, V6, whereas there is a single digital module 121, capable of treating, in rapid sequence, each of the analogic signals provided to it in input from the corresponding analogue module, ensuring an excellent simultaneity factor between the signals of the different leads. The acquisitions occur in sequence with a time delay between two consecutive leads of less than about 25 us: consequently, the eight leads are treated in a time of less than 200 us.

The analogue module 101 treats the signal in analogue mode, has one chain for each of the eight signals under examination, and includes the:

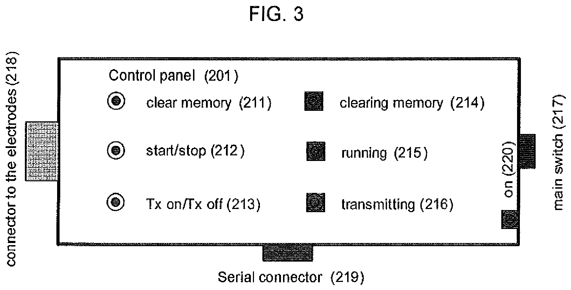

front-end submodule towards the electrodes: the submodule 111 is directly connected to screened cables that connect the acquisition subsystem to the electrodes arranged on the body surface of the patient (see also the connector 218 in Figure 3) through high-value resistances (100 Kohm). The front-end submodule carries out the impedance adaptation between the acquisition subsystem and the electrodes, as well as the generation of the reference/active mass level and the screening from disturbances coming from the outside;

preamplification and low pass (LP) filtering submodule: the submodule 112 carries out a first analogue preamplification of the signal and low pass filtering, eliminating the undesired components at high frequency (above 150 Hz, preferably above 200 Hz) so as to allow correct sampling at 500 Hz with rejection of image frequencies (anti aliasing). The filtering allows most of the disturbances detected by the upstream chain (cables, electrodes, connectors) to be eliminated;

amplification and high pass (HP) filtering submodule: the submodule 113 carries out a further amplification of the signal and removes the components at the lowest frequency (up to 0.25 Hz, preferably up to 0.15 Hz), like for example the oscillations of the average value and/or of the base line due to breathing.

The digital module 121 carries out an analogue-digital conversion of the input supplied signals, stores them locally and transmits them to the outside through a serial communication. The digital module is made up of the following submodules:

mux (multiselector or multiplexer): such a submodule 131 makes it possible to selectively connect the outputs from the analogue submodules of the single leads to the input of the digital module;

analogue/digital (A/D) converter: such a submodule 132 provides for the analogue/digital (A/D) conversion of the signals of the eight leads considered and acquired directly D1, D2, V1, V2, V3, V4, V5, V6. The conversion takes place at a sampling frequency greater than or equal to 400 Hz, advantageously at 500 Hz. The quantization takes place on 4096 distinct levels (12 significant bits). The simultaneity factor is such as to limit the maximum value of the time distance between the samples acquired and treated in the first and in the last lead;

memory: such a submodule 133 is made up of memories able to maintain its own content even in the absence of power, like for example RAM memories with buffer battery, EPROM, EAROM, Flash or any other technology useful for the purpose, The memory is able to memorise the 12 bit samples of the eight leads acquired at 500 Hz. The size of the memory submodule determines the maximum duration of the recording that can be made. The memory is able to continue to store its own content even in the case of removal or depletion of the power supply batteries, preserving the content of the recording;

serial transmission: the submodule 134 allows the serial transmission of the samples acquired. Such transmission can take place by directly connecting a peripheral RS232 to the serial connector 219 (see Fig. 3), or by connecting a RS232-Bluetooth adapter to the serial connector 219;

CPU: such a submodule 135 manages the entire digital part, controlling the A/D converter 132, the memory 133 and the reading/writing operations, the control buttons, the switching on of the light indicators, the serial transmission. As an alternative to the submodule CPU 135, the system can adopt a suitable microcontroller submodule, capable of carrying out the functions of the mux 131, A/D converter 132 and CPU 135 submodules in integrated mode.

The shell of the acquisition subsystem is very small in size: 150x80x50 millimetres. The upper surface of the shell houses the control panel 201 shown in Figure 3 with the following buttons, corresponding to the various activities:

main switch: the switch 217 switches the entire acquisition subsystem on/off, by acting on the power supply provided by three AA batteries of 1.5 Volt each. The light indicator 220 is switched on when the acquisition subsystem is activated;

"clear memory": the button 211 allows the content of the memory 112 to be erased, setting the acquisition subsystem for another acquisition. The time necessary to erase the content of the memory depends on the size of the memory itself. During the memory erasing operation, the light indicator 214 ("cleaning") stays switched on;

"start/stop": the button 212 starts/stops the acquisition of the ECG signal. When the memory is full, the acquisition is forced to stop. During the acquisition of the signal, the light indicator 215 ("running") stays switched on;

"Tx on/Tx off": the button 213 starts/stops the transmission of the signals acquired and present in the memory. If the transmission is not in progress, "Tx on/Tx off" starts the transmission, always starting from the first sample acquired; if the transmission is in progress, "Tx on/Tx off" instantly stops the transmission of the samples; the transmission automatically stops when all of the samples have been transmitted. In order to activate the "monitor" operating mode, i.e. acquisition, storage and simultaneous transmission of the ECG signal on the serial channel, it is necessary to press in sequence the buttons "clear memory" 211, "Tx on/Tx off 213, "start/stop" 212: the end of the "monitor" operating mode occurs by pressing the "start/stop" button or when the memory is full. During transmission the light indicator 216 ("transmitting") stays switched on.

Further characteristics concern the low energy consumption, which allows the acquisition subsystem to operate uninterruptedly for over 36 hours.

The size of the memory 133 defines the maximum duration of a recording, The hourly memory consumption is obtained as follows:

2byte/sample x 500 samples/sec x 8 leads x

$$60 \text{ sec/minute} \times 60 \text{ minutes/hour} = 27.5 \text{ Mbyte/hour}$$

Using a memory of 1 Gigabyte, the maximum duration of a recording is over 30 hours. From what has been outlined the advantages of the electrocardiographic acquisition subsystem according to the invention are clear, having extremely small dimensions, being easy and intuitive to use, with professional characteristics, capable of providing all twelve of the clinical-interesting leads.

**Proximal elaborating subsystem**

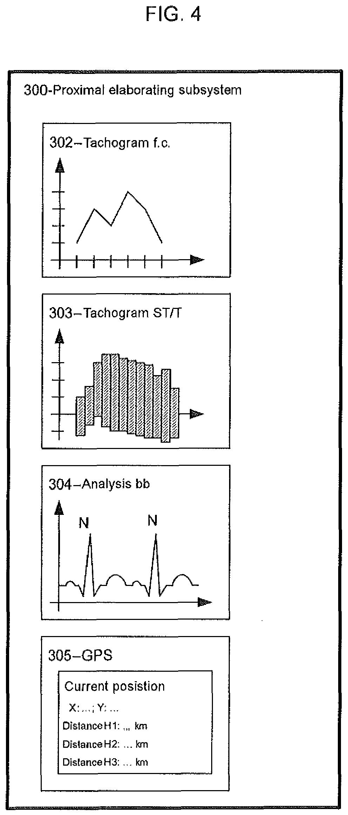

The proximal elaborating subsystem 300 has information both in text form and in graphical form (Fig.4).

The information in text form concerns the personal data of the patient (Surname, Forename, date of birth), the current date and time: a panic button allows a message to be sent immediately to the mobile telephone of the doctor with the URL to gain access to the trace that the proximal elaborating subsystem has sent to the dedicated Web server and relative to the last 15 minutes of electrocardiographic recordings. In this way, the doctor can remotely check the possible electrocardiographic variations undergone by the subject. In order to ensure suitable privacy, the doctor will have to insert a suitable enabling password in order to be able to access the functions of the dedicated Web server.

The information in graphical form concerns the tachogram of the heart rate 302, the tachogram of the areas ST-T 303, the displaying of the trace and the beat-beat analysis with the classification of each single beat 304.

The tachogram of the heart rate 302 shows the number of the beat on the horizontal axis and the instantaneous cardiac frequency measured as the distance between the current beat and the previous beat on the vertical axis.

The tachogram of the areas ST-T 303 shows the number of the beat on the horizontal axis and the value of the area subtended, during the portion ST-T, by the electrocardiographic signal with respect to the base line on the vertical axis.

The displaying of the trace shows, for each beat, the classification of the same beat according to the annotations provided by the signals archive "MIT/BIH Arrhythmia Database": e.g., the annotation "N" indicates that the beat was as normal. The displaying of the signal takes place in left scroll mode (when the displaying of the trace has reached the right margin of the display, the arrival of a new sample of the signal generates a scrolling to the left of the trace displayed previously) or in static mode (when the displaying of the trace has reached the right margin of the display, the displaying starts back from the left margin of the display, without carrying out the scrolling of the trace itself but overwriting the pre-existing trace).

The information in text form concerns the calculation of the current GPS position as well as the calculation of the distance of the current position from points of interest whose GPS coordinates have been stored in advance 305.

The invention is defined by the attached claims.

**Claims**

1. Portable and wearable system for the acquisition, storing, elaboration, real-time proximal local displaying, analysis, and transmission of electrocardiographic signals, adapted to permit the evaluation of all twelve clinical leads comprising:

   - an analogue module (101) for each of the eight clinical leads D1, D2, V1, V2, V3, V4, V5, V6 directly acquisable through electrodes; and
   - a digital module (121) able to process in sequence the analogic signals supplied at the inlet by the analogue modules (101);
   - a proximal local displaying and elaborating subsystem (300) configured to reconstruct the other four clinical-interesting leads D3, aVr, aV1, aVf starting from the directly acquired leads D1, D2, V1, V2, V3, V4, V5, V6;

   each analogue module (101) comprising:

   - a front-end submodule (111) adapted to be connected to the electrodes placed on the body surface of the patient;

   a preamplification and low-pass filtering submodule (112), adapted to perform an analogue preamplification of the signal and to remove the undesired components at high frequency;
   an amplification and high-pass filtering submodule (113) adapted to perform a further amplification of the signal and to remove the components at lower frequency;
   the digital module (121) comprising:

   a multiplexer submodule (131) adapted to permit the selective connection of the outputs from the analogue modules (101) of the single leads to the inlet of the digital module (121);
   an analogue/digital A/D converter submodule (132);
   a memory submodule (133);
   a transmitting submodule (134);
   a control submodule (135), defining a CPU, adapted to manage the other submodules, by controlling the A/D converter (132), the memory (133) and the transmission,

wherein
the sampling frequency of the A/D submodule (132) is being greater than or equal to 400 Hz, preferably greater than or equal to 500 Hz; such to maintain in the digitalized signal the whole information present in the analogue electrocardiographic signal acquired from said clinical leads, both in case of ischaemic pathologies and other pathologies including the arrhythmic ones, supplying characteristics in the processing of the signals complying with the requirements of the professional systems and used in the clinical/hospital field;
all the 12 leads used in the clinical practice are recorded simultaneously so as to permit the simultaneous analysis of the trace observed by more leads;
the proximal local elaborating subsystem (300) being adapted to:

- display in real-time the electrocardiographic signals;
- calculate the instantaneous cardiac frequency;
- perform an elementary analysis for automatically classifying the arrhythmic and/or ischaemic alterations, also without the transmission to a remote centre of elaboration, thus displaying the results of the analysis even in case of no remote connection;
transmit the signals to a remote centre for remote distal analysis by specialists;
- calculate the actual position of the patient through a GPS system;
- calculate the distance from the actual position to specific points previously stored, such as hospitals, first aid centres or assistance centres, thus allowing the operator or the subject himself to immediately identify the nearest hospital or care centre.

2. System according to claim 1, wherein said proximal elaborating subsystem (300) belongs to the group including PC, tablet PC, tablet, iPad, mobile telephone, smart-phone.

3. System according to claim 1 or 2, wherein all of the functionalities of the elaboration subsystem (300) are adapted to operate also in the absence of remote connections of the group comprising telephonic wires, mobile telephone signal (telephonic manager), internet, WiFi.

4. System according to any one of the previous claims, wherein the quantization of the signal samples made by the analogue/digital A/D converter submodule (132) is at least 1024 levels, i.e. on at least 10 significant bits, preferably 4096 levels, i.e. 12 significant bits.

5. System according to any one of the previous claims, wherein the preamplification and low-pass filtering submodule (112) is such to remove the signal components at high frequency over 150 Hz, preferably over 200 Hz and the amplification and high-pass filtering submodule (113) removes the components with frequency of less than 0.25 Hz of the signal, preferably less than 0.15 Hz, such as the oscillations of the average value and/or of the base line due to breathing.

6. System according to any one of the previous claims, wherein said memory submodule (133) is formed by memories able to store their own content even in the absence of power, such as the memories of the group comprising RAM memories having buffer battery, EPROM, EAROM, Flash, adapted to permit a maximum recording duration of over 30 hours.

7. System according to any one of the previous claims, wherein the transmitting submodule performs a serial-type transmission, with a protocol of the group comprising RS232, Bluetooth, USB or WiFi.

8. System according to any one of the previous claims, wherein said control submodule (135), said multiplexer submodule (131) and said A/D submodule converter (132) are integrated in a microcontroller unit.

9. System according to any one of the previous claims equipped with a control panel (201) and battery power supply.

10. System according to any one of the previous claims, wherein said analogue modules (101), said digital module (121) and the control panel (201) are housed in a shell having dimensions not greater than 150x80x80 mm, and have a total weight not greater than 300 grams.

11. System according to any one of claims 9, 10, wherein said control panel comprises:

a main switch (217) adapted to turn on/off the whole system acting on the electrical power supplied by the batteries, the state of which is indicated by a light indicator (220);

a "clear memory" button (211) adapted to erase the content of the memory (112) in order to set the system for a new acquisition, the state of which is indicated by a light indicator (214) which is lighted during the erasing of the memory;

a "start/stop" button (212) adapted to start/stop the acquisition of the ECG signal, the state of which is indicated by a light indicator (215) which is lighted during the acquisition of the signal;

a "Tx on/Tx off" button (213) adapted to start/stop the transmission of the signals acquired and present in the memory, the state of which is indicated by a light indicator (216) which is lighted during the transmission.

12. System according to claim 11, wherein said "Tx on/Tx off" button (213) is adapted to start the transmission, starting always from the first acquired sample, and said "Tx on/Tx off" button is adapted to instantaneously suspend the transmission of the samples, the transmission being stopped automatically when all the samples are transmitted.

13. System according to claim 11 or 12 adapted to activate the functioning mode "monitor", that is the acquisition, the memorization and the simultaneous transmission of the ECG signal on the serial channel, pushing in sequence the buttons "clear memory" (211), "Tx on/Tx off" (213), "start/stop" (212), and adapted to stop the functioning mode "monitor" by pushing the button "start/stop" (212) or at the saturation of the memory (133).

14. System according to any one of the previous claims comprising 10 electrodes.


**Patentansprüche**

1. Tragbares und anziehbares System für die Ermittlung, Speicherung, Erarbeitung, lokale proximale Echtzeit-Anzeige, Analyse und Übertragung elektrokardiographischer Signale, das dazu angepasst ist, die Bewertung aller zwölf klinischen Kabel zu erlauben, umfassend:

- ein analoges Modul (101) für jedes der acht klinischen Kabel D1, D2, V1, V2, V3, V4, V5, V6, die durch Elektroden direkt ermittelbar sind; und
- ein digitales Modul (121), das fähig ist, in Abfolge die analogen Signale zu verarbeiten, die an dem Einlass durch die analogen Module (101) geliefert werden;
- ein lokales proximales Anzeige- und Erstellungssubsystem (300), das konfiguriert ist, um die anderen vier Kabel D3, aVr, aV1, aVf von klinischem Interesse ausgehend von den direkt ermittelten Kabeln D1, D2, V1, V2, V3, V4, V5, V6 zu rekonstruieren;

wobei jedes analoge Modul (101) umfasst:

- ein Frontend-Submodul (111), das dazu angepasst ist, mit den Elektroden verbunden zu sein, die auf der Körperoberfläche des Patienten platziert werden;

ein Vorverstärkungs- und Tiefpassfilter-Submodul (112), das dazu angepasst ist, eine analoge Vorverstärkung des Signals auszuführen und die unerwünschten Komponenten mit hoher Frequenz zu entfernen;

ein Verstärkungs- und Hochpassfilter-Submodul (113), das dazu angepasst ist, eine weitere Verstärkung des Signals auszuführen und die Komponenten mit niedrigerer Frequenz zu entfernen;

wobei das digitale Modul (121) umfasst:

ein Multiplexer-Submodul (131), das dazu angepasst ist, die selektive Verbindung der Ausgänge aus den analogen Modulen (101) der einzelnen Kabel mit dem Einlass des digitalen Moduls (121) zu erlauben;
ein Analog-/Digital-A/D-Wandler-Submodul (132);
ein Speichersubmodul (133);
ein Übertragungssubmodul (134);
ein Steuersubmodul (135), das eine CPU definiert, die angepasst ist, die anderen Submodule durch Steuern des A/D-Wandlers (132), des Speichers (133) und der Übertragung zu verwalten, wobei
die Abtastfrequenz des A/D-Wandlers (132) größer oder gleich 400 Hz ist, vorzugsweise größer oder gleich 500 Hz; um in dem digitalisierten Signal die gesamten Informationen aufrechtzuerhalten, die in dem analogen elektrokardiographischen Signal anwesend sind, die aus den klinischen Kabeln, sowohl in dem Fall ischämischer Pathologien als auch anderer Pathologien, einschließlich der arrhythmischen, ermittelt werden, sowohl Merkmale bei der die Verarbeitung der Signale zu liefern, die mit den Anforderungen der professionellen Systeme übereinstimmen als auch im klinischen/Krankenhausumfeld verwendet werden;
alle 12 Kabel, die in der klinischen Praxis verwendet werden, gleichzeitig aufzunehmen, um die gleichzeitige Analyse des Verlaufs, der von mehreren Kabeln beobachtet wird, zu erlauben;
wobei das lokale proximale Erstellungssubsystem (300) dazu angepasst ist:

- in Echtzeit die elektrokardiographischen Signale anzuzeigen;
- die Momentan-Herzfrequenz zu berechnen;
- eine elementare Analyse zum automatischen Klassifizieren der arrhythmischen und/oder ischämischen Veränderungen auch ohne die Übertragung zu einem entfernten Erstellungszentrum auszuführen, so dass die Resultate der Analyse sogar in dem Fall ohne Fernverbindung angezeigt werden;
die Signale zu einem Fernzentrum für distale Fernanalyse durch Spezialisten zu übertragen;
- die aktuelle Position des Patienten durch ein GPS-System zu berechnen;
- die Entfernung von der aktuellen Position zu spezifischen Punkten, die vorab gespeichert werden, wie zu Krankenhäusern, Erste-Hilfe-Zentren oder Hilfszentren, zu berechnen, so dass es dem Bediener oder dem Subjekt selbst erlaubt wird, das nächstgelegene Krankenhaus oder Pflegezentrum sofort zu identifizieren.

2. System nach Anspruch 1, wobei das proximale Erstellungssubsystem (300) zu der Gruppe gehört, die einen PC, Tablet-PC, ein Tablet, einen iPad, ein Mobiltelefon, ein Smartphone beinhaltet.

3. System nach Anspruch 1 oder 2, wobei alle der Funktionalitäten des Erstellungssubsystems (300) dazu angepasst sind, auch bei Abwesenheit von Fernverbindungen der Gruppe, die Telefonleitungen, Mobiltelefonsignal (Telefonmanager), Internet, WiFi umfasst, zu arbeiten.

4. System nach einem der vorstehenden Ansprüche, wobei die Quantisierung der Signalproben, die von dem Analog/Digital-A/D-Wandler (132) hergestellt werden, mindestens 1024 Niveaus beträgt, das heißt auf mindestens 10 signifikanten Bits, vorzugsweise 4096 Niveaus, das heißt 12 signifikanten Bits.

5. System nach einem der vorstehenden Ansprüche, wo bei das Vorverstärkungs- und Tiefpassfilter-Submodul (112) derart ist, dass es die Signalkomponenten mit hoher Frequenz über 150 Hz, vorzugsweise über 200 Hz, entfernt, und das Verstärkungs- und Hochpassfilter-Submodul (113) die Komponenten mit einer Frequenz von weniger als 0,25 Hz des Signals, vorzugsweise weniger als 0,15 Hz, wie die Oszillationen des Mittelwerts und/oder der Grundlinie aufgrund von Atmen, entfernt.

6. System nach einem der vorstehenden Ansprüche, wobei das Speichersubmodul (133) aus Speichern gebildet ist, die fähig sind, ihren eigenen Inhalt sogar bei Abwesenheit von Strom zu speichern, wie die Speicher der Gruppe, die RAM-Speicher, die Pufferbatterien aufweisen, EPROM, EAROM, Flash, die dazu angepasst sind, eine maximale Aufzeichnungsdauer von über 30 Stunden zu erlauben.

7. System nach einem der vorstehenden Ansprüche, wobei das Übertragungssubmodul eine Übertragung des seriellen Typs mit einem Protokoll der Gruppe, die RS232, Bluetooth, USB oder WiFi umfasst, ausführt.

8. System nach einem der vorstehenden Ansprüche, wobei das Steuersubmodul (135), das Multiplexer-Submodul (131) und das A/D-Wandlersubmodul (132) in eine Mikrocontrollereinheit integriert sind.

9. System nach einem der vorstehenden Ansprüche, das mit einem Bedienfeld (201) und Batteriestromversorgung ausgestattet ist.

10. System nach einem der vorstehenden Ansprüche, wobei das analoge Modul (101), das digitale Modul (121) und das Bedienfeld (201) in einer Umhausung aufgenommen sind, die Maße aufweist, die nicht größer sind als 150 x 80 x 80 mm, und ein Gesamtgewicht von nicht mehr als 300 Gramm aufweist.

11. System nach einem der Ansprüche 9, 10, wobei das Bedienfeld umfasst:

einen Hauptschalter (217), der dazu angepasst ist, das gesamte System durch Einwirken auf den Strom, der von den Batterien geliefert wird, deren Zustand durch eine Leuchtanzeige (220) angegeben wird, ein-/auszuschalten;
einen "Speicherlöschen"-Knopf (211), der dazu angepasst ist, den Inhalt des Speichers (112) zu löschen, um das System für eine neue Ermittlung einzustellen, dessen Zustand durch eine Leuchtanzeige (214) angegeben wird, die während des Löschens des Speichers leuchtet;
einen "Start-/Stopp"-Knopf (212), der dazu angepasst ist, die Ermittlung des EKG-Signals zu starten/zu stoppen, dessen Zustand durch eine Leuchtanzeige (215) angegeben wird, die während der Ermittlung des Signals leuchtet,
einen "Tx On/Tx off"-Knopf (213), der dazu angepasst ist, die Übertragung der ermittelten Signale und die in dem Speicher anwesend sind, zu starten/zu stoppen,
dessen Zustand durch eine Leuchtanzeige (216) angegeben wird, die während der Übertragung leuchtet.

12. System nach Anspruch 11, wobei der "Tx On/Tx off"-Knopf (213) dazu angepasst ist, die Übertragung zu starten, immer beginnend ab der ersten ermittelten Probe, und der "Tx On/Tx off"-Knopf dazu angepasst ist, die Übertragung der Proben sofort zu unterbrechen, wobei die Übertragung automatisch gestoppt wird, wenn alle Proben übertragen wurden.

13. System nach Anspruch 11 oder 12, das dazu angepasst ist, den Betriebsmodus "Monitor" zu aktivieren, der die Ermittlung, die Speicherung und die gleichzeitige Übertragung des EKG-Signals auf dem seriellen Kanal ist, indem nacheinander die Knöpfe "Speicherlöschen" (211), "Tx On/Tx off" (213), "Start/Stopp" (212) gedrückt werden, und dazu angepasst ist, den Betriebsmodus "Monitor" zu stoppen, indem der Knopf "Start/Stopp" (212) gedrückt wird oder bei der Sättigung des Speichers (133).

14. System nach einem der vorstehenden Ansprüche, das 10 Elektroden umfasst.

**Revendications**

1. Système portable et mettable pour l'acquisition, le stockage, l'élaboration, l'affichage local proximal en temps réel, l'analyse et la transmission de signaux électrocardiographiques, adapté pour permettre l'évaluation des douze dérivations cliniques, comprenant :

- un module analogique (101) pour chacune des huit dérivations cliniques D1, D2, V1, V2, V3, V4, V5, V6 qui peuvent être acquises directement via des électrodes ; et
- un module numérique (121) capable de traiter séquentiellement les signaux analogiques fournis en entrée par les modules analogiques (101) ;
- un affichage local proximal et un sous-système d'élaboration (300) configuré pour reconstruire les quatre autres dérivations d'intérêt clinique D3, aVr, aVl, aVf en commençant par les dérivations directement acquises D1, D2, V1, V2, V3, V4, V5, V6 ;

chaque module analogique (101) comprenant :

- un sous-module frontal (111) adapté pour être connecté aux électrodes placées sur la surface du corps du patient ;

un sous-module de préamplification et de filtration passe-bas (112), adapté pour mettre en œuvre une préamplification analogique du signal et pour éliminer les composantes haute fréquence indésirables ;
un sous-module d'amplification et de filtration passe-haut (113) adapté pour mettre en œuvre une amplification additionnelle du signal et pour éliminer les composantes basse fréquence ;
le module numérique (121) comprenant :

un sous-module multiplexeur (131) adapté pour permettre une connexion sélective des sorties provenant des modules analogiques (101) des dérivations individuelles à l'entrée du module numérique (121) ;
un sous-module convertisseur analogique/numérique A/N (132) ;
un sous-module de mémoire (133) ;
un sous-module de transmission (134) ;
un sous-module de contrôle (135), qui définit un processeur, adapté pour gérer les autres sous-modules en contrôlant le convertisseur A/N (132), la mémoire (133) et la transmission, dans lequel
la fréquence d'échantillonnage du sous-module A/N (132) est supérieure ou égale à 400 Hz, et de préférence supérieure ou égale à 500 Hz, de manière à maintenir, dans le signal numérisé, toute l'information présente dans le signal électrocardiographique analogique acquis via lesdites dérivations cliniques, aussi bien dans le cas de pathologies ischémiques que dans le cas d'autres pathologies, y compris les arythmies, en alimentant

**EP 2 526 859 B1**

lors du traitement des signaux des caractéristiques conformes aux exigences des systèmes professionnels et utilisées dans le domaine clinique/hospitalier ;

toutes les 12 dérivations utilisées en usage clinique sont enregistrées simultanément de manière à permettre l'analyse simultanée de la trace observée par d'autres dérivations ;

le sous-système d'élaboration local proximal (300) étant adapté pour :

- afficher en temps réel les signaux électrocardiographiques ;
- calculer la fréquence cardiaque instantanée ;
- mettre en œuvre une analyse élémentaire pour classifier automatiquement les altérations arythmiques et/ou ischémiques, aussi sans transmission à un centre d'élaboration distant, affichant ainsi les résultats de l'analyse même en cas d'absence de connexion à distance ;
- transmettre les signaux à un centre distant pour une analyse à distance par des spécialistes ;
- calculer la position actuelle du patient via un système GPS ;
- calculer la distance entre la position actuelle et des points spécifiques enregistrés préalablement, tels que des hôpitaux, des centres de premiers secours et des centres d'assistance, permettant ainsi à l'opérateur ou au sujet lui-même d'identifier immédiatement l'hôpital ou le centre de soins le plus proche.

2. Système selon la revendication 1, dans lequel ledit sous-système d'élaboration proximal (300) appartient au groupe comprenant un ordinateur personnel, une tablette, un iPad, un téléphone portable et un téléphone intelligent.

3. Système selon la revendication 1 ou 2, dans lequel toutes les fonctionnalités du sous-système d'élaboration (300) sont adaptées pour fonctionner aussi en l'absence de connexions distantes appartenant au groupe comprenant une connexion téléphonique filaire, un signal de téléphone mobile (gestionnaire téléphonique), internet, et une connexion Wi-Fi.

4. Système selon l'une quelconque des revendications précédentes, dans lequel la quantification des échantillons de signal effectuée par le sous-module convertisseur analogique/numérique A/N (132) est d'au moins 1024 niveaux, c'est-à-dire d'au moins 10 bits significatifs, et de préférence de 4096 niveaux, c'est-à-dire de 12 bits significatifs.

5. Système selon l'une quelconque des revendications précédentes, dans lequel le sous-module de préamplification et de filtration passe-bas (112) permet d'éliminer les composantes de signal haute fréquence supérieures à 150 Hz, et de préférence supérieures à 200 Hz, et le sous-module d'amplification et de filtration passe-haut (113) élimine les composantes du signal à fréquence inférieures à 0,25 Hz, et de préférence inférieures à 0,15 Hz, telles que les oscillations de la valeur moyenne et/ou de la ligne de base dues à la respiration.

6. Système selon l'une quelconque des revendications précédentes, dans lequel ledit sous-module de mémoire (133) est constitué de mémoires capables de stocker leur propre contenu même en l'absence d'alimentation d'énergie, telles que des mémoires appartenant au groupe comprenant les mémoires vives ayant une pile tampon, les mémoires EPROM, les mémoires EAROM et les mémoires flash, permettant une durée d'enregistrement maximale supérieure à 30 heures.

7. Système selon l'une quelconque des revendications précédentes, dans lequel le sous-module de transmission met en œuvre une transmission de type série, avec un protocole appartenant au groupe comprenant les protocoles RS232, Bluetooth, USB et Wi-Fi.

8. Système selon l'une quelconque des revendications précédentes, dans lequel ledit sous-module de contrôle (135), ledit sous-module multiplexeur (131) et ledit sous-module convertisseur A/N (132) sont intégrés dans une unité de microcontrôleur.

9. Système selon l'une quelconque des revendications précédentes, équipé d'un panneau de contrôle (201) et d'une alimentation par pile.

10. Système selon l'une quelconque des revendications précédentes, dans lequel lesdits modules analogiques (101), ledit module numérique (121) et le panneau de contrôle (201) sont logés dans une coque dont les dimensions ne dépassent pas 150 x 80 x 80 mm, et dont la masse totale ne dépasse pas 300 grammes.

11. Système selon l'une quelconque des revendications 9, 10, dans lequel ledit panneau de contrôle comprend :

un commutateur principal (217) adapté pour allumer/éteindre tout le système en agissant sur l'alimentation électrique fournie par les piles, dont l'état est indiqué par un témoin lumineux (220) ;

un bouton « effacer la mémoire » (211) adapté pour effacer le contenu de la mémoire (112) de manière à configurer le système pour une nouvelle acquisition, dont l'état est indiqué par un témoin lumineux (214) qui est allumé durant l'effacement de la mémoire ;

un bouton « démarrage/arrêt » (212) adapté pour démarrer/arrêter l'acquisition du signal ECG, dont l'état est indiqué par un témoin lumineux (215) qui est allumé durant l'acquisition du signal ;

un bouton « Tx actif/Tx inactif » (213) adapté pour démarrer/arrêter la transmission des signaux acquis et présents en mémoire, dont l'état est indiqué par un témoin lumineux (216) qui est allumé durant la transmission.

12. Système selon la revendication 11, dans lequel ledit bouton « Tx actif/Tx inactif » (213) est adapté pour démarrer la transmission, en commençant toujours par le premier échantillon acquis, et ledit bouton « Tx actif/Tx inactif » est adapté pour suspendre instantanément la transmission des échantillons, la transmission étant arrêtée automatiquement quand tous les échantillons sont transmis.

13. Système selon la revendication 11 ou 12, adapté pour activer le mode de fonctionnement « moniteur », qui correspond à l'acquisition, à la mémorisation et à la transmission simultanée du signal ECG sur le canal série, en poussant séquentiellement sur les boutons « effacer la mémoire » (211), « Tx actif/Tx inactif » (213), « démarrage/arrêt » (212), et adapté pour arrêter le mode de fonctionnement « moniteur » en poussant sur le bouton « démarrage/arrêt » (212) ou lorsque la mémoire (133) est saturée.

14. Système selon l'une quelconque des revendications précédentes, comprenant 10 électrodes.

# FIG. 1

500 – Entire system

| 100 Acquisition subsystem | → | 300 Proximal elaborating subsystem | → | Remote elaboration |

# FIG. 2

From electrodes

Analog module (101)

| (111) Front-end electrodes | → | (112) Pre-amplification and LP filter | → | (113) Amplification and HP filter | → |

Digital module (121)

| (131) mux | → | (132) A / D Converter | → | (133) Memory | → | (134) Serial transmission |

(135) CPU

(141) Transmission module

To the proximal elaborating subsystem ( External devices for displaying, buffering, elaboration, remote transmission )

FIG. 3

Control panel (201)

| | | | |
|---|---|---|---|
| ⊙ | clear memory (211) | ▪ | clearing memory (214) |
| ⊙ | start/stop (212) | ▪ | running (215) |
| ⊙ | Tx on/Tx off (213) | ▪ | transmitting (216) |

connector to the electrodes (218)

main switch (217)

on (220)

Serial connector (219)

# FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5704351 A, Mortara
- US 5755230 A, Schmidt
- US 20020115914 A1
- US 2004109992 A1
- EP 1629769 A1
- EP 1803392 A2
- EP 2082683 A1
- EP 2000085 A2, Politecnico di Milano
- EP 1188412 A2

- WO 2004030756 A1
- EP 0673233 A
- US 5876351 A
- US 20070208262 A
- US 20070208232 A
- US 20070208233 A
- US 6970737 B
- US 8763351 A

**Non-patent literature cited in the description**

- A Multiparameter Wearable Physiologic Monitoring System for Space and terrestrial Applications. *IEEE TITB,* September 2005, vol. 9 (3